(19) Europäisches Patentamt
European Patent Office
Office européen des brevets

(11) Publication number: 0 028 921
A1

# (12) EUROPEAN PATENT APPLICATION

(21) Application number: 80303962.7

(51) Int. Cl.[3]: C 07 D 213/80

(22) Date of filing: 06.11.80

(30) Priority: 09.11.79 US 92795

(43) Date of publication of application: 20.05.81 Bulletin 81/20

(84) Designated Contracting States: CH DE FR GB LI

(71) Applicant: UNIROYAL, INC.
1230 Avenue of the Americas Rockefeller Center
New York, New York 10020(US)

(72) Inventor: Doweyko, Arthur M.
99-6 Ridge Road Naugatuck
New Haven Connecticut(US)

(74) Representative: Harrison, Michael Robert et al,
URQUHART-DYKES & LORD 11th Floor Tower House
Merrion Way
Leeds LS2 8PB(GB)

## (54) Alkylation of sulfones.

(57) The alkylation of substituted 2-sulfonyl pyridine 1-oxides is brought about by the admixing of the compound to be alkylated, a polar aprotic solvent, a strong base and an alkylating agent.

EP 0 028 921 A1

5687

# ALKYLATION OF SULFONES

Certain aryl sulfonyls are alkylated through use of a polar aprotic solvent, a strong base and alkylating agents.

U.S. Patent No. 3,960,542, issued June 1, 1976, discloses, inter alia, novel substituted 2-sulfonyl pyridine 1-oxides useful as herbicides. A method of controlling weeds through use of such compounds is disclosed in U.S. Patent No. 4,019,893, issued April 16, 1977. The disclosures of both of the aforementioned patents are herewith incorporated by reference herein. As taught in both of these patents, the subject compounds may be prepared via formation of intermediate thio compounds, i.e., substituted 2-thiopyridines, and the subsequent oxidation of same to substituted 2-sulfonyl pyridine 1-oxides. Alternatively, the 2-sulfonyl pyridine 1-oxide derivatives may be prepared from known compounds. See, for example, W. Walter et al., Liebig's Ann., 695, 77 (1966); E. Shaw et al., J.A.C.S. 72, 4362 (1050); A. R. Katritsky, J. Chem. Soc., 191 (1957); and U.S. Patents Nos. 3,107,994 and 3,155,671. Parent 2-thiopyridine 1-oxides may be prepared by (1) the reaction of 2-chloropyridine 1-oxide with the appropriate mercaptan in the presence of an acid acceptor such as an alkaline earth hydroxide; or (2) reaction of the sodium salt of 2-mercaptopyridine 1-oxide with a suitable halide preferentially, but not necessarily, of the benzyl type.

Normally, compounds of the type here relevant may be alkylated through use of a very strong base such as, for example, sodium hydride or sodium amide. But these compounds are extremely reactive, result in the formation of troublesome by-products such as hydrogen or ammonia, and must be employed only under very anhydrous conditions requiring, for example, use of an inert atmosphere.

An object of the invention is to permit alkylation of substituted 2-sulfonyl pyridine 1-oxides while reducing or avoiding the problems associated with other means of alkylating such compounds.

It has now been found that substituted 2-sulfonyl pyridine 1-oxides may be alkylated via an alkylating agent in a polar aprotic solvent and a strong base.

More specifically, starting materials may be selected from the substituted 2-sulfonyl pyridine 1-oxides having the general form

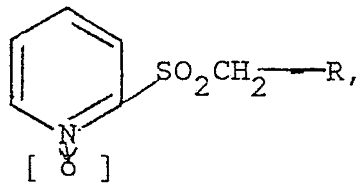

wherein R may be 2,2-dichlorocyclopropyl, 2,2-dichloro-1-methylcyclopropyl, naphthyl, methylnaphthyl, monosubstituted phenyl wherein the substituent is in the ortho position and is selected from alkyl having 1 to 2 carbon atoms, halogen or nitro, or polysubstituted phenyl having from 2 to 5 substituents which may be the same or different and are selected from alkyl having 1 or 2 carbon atoms, halogen, nitro, methoxy, ethoxy and methylenedioxy. Of special interest are those substituted 2-sulfonyl pyridine 1-oxides wherein R is selected from the group consisting of phenyl, phenyl substituted with 1 or 2 methyl groups, monohalophenyl and 2-napthyl, especially 2-methylphenyl, 3-methylphenyl and 2,5-dimethylphenyl. A preferred starting material is 2-(2,5-dimethylphenylmethylsulfonyl) pyridine 1-oxide.

Representative of alkylating agents useful in this invention are alkyl and substituted alkyl halides and sulfates. Particularly interesting alkyl and substituted alkyl halides include the methyl, benzyl and allyl halides and substituted methyl, benzyl and naphthyl halides, respectively. Preferred alkylating agents for use herein include methyl chloride, benzyl chloride, propargyl bromide, dimethyl sulfate, methyl iodide, ethyl bromide, 3-trifluoromethylbenzyl chloride, allyl chloride, vinyl sulfone, 1,3-dibromopropane, 1,6-dibromohexane and 2-chloromethylnapthalene, with methyl chloride, benzyl chloride, propargyl bromide and dimethyl sulfate being most preferred.

Polar aprotic solvents particularly useful herein include N,N-dimethylformamide, N,N-dimethylacetamide and dimethylsulfoxide. Aprotic solvents of lesser polarity than the just-named compounds, such as methylene chloride and acetonitrile, are also useful but result in drastically lower yields.

Strong bases that can be used include the alkaline earth hydroxides, with sodium hydroxide being especially preferred.

Alkylations according to the invention yield products having the general form

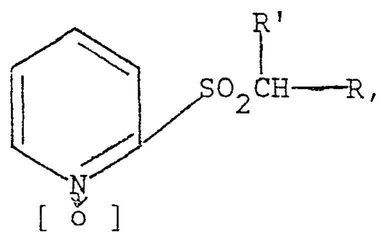

where R is as defined above and R' may be alkyl or substituted alkyl. Preferably R' is selected from the group consisting of methyl, ethyl, benzyl and substituted methyl, benzyl and naphthyl such as 2-propenyl, 2-propynyl, 3-trifluoromethylbenzyl and 2-1-methylnaphthyl. Most preferably, R' is selected from the group consisting of methyl, benzyl and 2-propynyl.

As presently understood, the use of a polar aprotic solvent is essential to this process due to such solvent's enhancement of the basicity of the strong base used.

Generally, the substituted 2-sulfonyl pyridine 1-oxide to be alkylated is admixed with a polar aprotic solvent, a strong base and an alkylating agent to yield the desired alkylated substituted 2-sulfonyl pyridine 1-oxide. Preferably, the substituted 2-sulfonyl pyridine 1-oxide to be alkylated is added to a solution comprising the solvent, strong base and alkylating agent.

In carrying out alkylations according to the invention as shown in the following examples, the polar aprotic solvent used was N, N-dimethylformamide (used without drying); the strong base was freshly ground to a powder before use, with care being taken to avoid prolonged exposure to moisture; reaction temperatures were maintained in the range of from about 10°C to 40°C; and the reaction time varied with the specific alkylating agent employed.

Example I

To a well-stirred solution of 1.7 g (42.5 mmol) powdered sodium hydroxide and 2.66 g (21.1 mmol) dimethylsulfate in 10 mL of DMF was added 5.00 g (18.1 mmol) 2-(2,5-dimethylphenyl methyl sulfonyl) pyridine 1-oxide in one portion. The reaction temperature was maintained at 10°C for 10 minutes. The reaction mixture was

then poured into 300 mL of rapidly-stirred ice-cold water to afford a flocculent white precipitate. This suspension was filtered, washed with water, and air-dried to give 4.80 g of 2-[1-(2,5-dimethylphenyl) ethylsulfonyl] pyridine 1-oxide (91% pure by H-NMR analysis). This represents a 91% recovery, with an overall yield of 81%.

Example II

To a well-stirred solution of 40 g (1.0 mol) powdered sodium hydroxide and 102 g (2.04 mol) methyl chloride in 700 mL DMF was added 197 g (0.71 mol) 2-(2,5 -dimethylphenylmethyl sulfonyl) pyridine 1-oxide over a period of 5 minutes. The reaction temperature was maintained at 30-40°C over a period of 4 hours. The reaction mixture was then poured into 2 L of rapidly-stirred ice-cold water to afford a flocculent white precipitate. The precipitate was isolated by filtration, washed with water, and air dried to give 186 g (90% yield) of 2-[1-(2,5-dimethylphenyl) ethylsulfonyl] pyridine 1-oxide (99% pure by H-NMR analysis and HPLC).

Example III

To a well-stirred solution of 1.50 g (37.5 mmol) powdered sodium hydroxide and 4.1 g (32.4 mmol) benzyl chloride in 25 mL DMF was added 8.3 g (30.0 mmol) of 2-(2,5-dimethylphenylmethyl sulfonyl) pyridine 1-oxide in one portion. The temperature was maintained at 35°C for 45 minutes. The reaction mixture was poured into 300 mL of rapidly-stirred ice-cold water to afford a flocculent white precipitate which was filtered off, washed with water, and air dried to give 9.3 g of crude product. Crystallization from ethanol yielded 7.0 g (64% yield) of pure 2-[1-(2,5-dimethylphenyl)-2-phenylethylsulfonyl] pyridine 1-oxide, m.p. 175-178°C (IR Spectrum, $SO_2$, 1145, 1315 $cm^{-1}$).

Analysis: Calculated for $C_{21}H_{21}NO_3S$: C 68.66; H 5.72; N 3.81. Found: C 68.10; H 5.71; N 3.82.

Example IV

To a well-stirred solution of 1.80 g (45.0 mmol) of powdered sodium hydroxide and 5.4 g (45.4 mmol) propargyl bromide in 25 mL DMF was added 11.0 g (39.7 mmol) 2-(2,5-dimethylphenylmethyl sulfonyl) pyridine 1-oxide in one portion. The temperature reached

50°C within a few minutes and was immediately cooled and maintained at 30°C for 1 hour. The reaction mixture was poured into 300 mL of rapidly-stirred ice-cold water to afford a flocculent precipitate, which was filtered off, washed with water and air dried to give 10 g crude product. Crystallization from ethanol gave 8.0 g (64% yield) of pure 2-[ x-(2,5-dimethylphenyl)- x-propargyl methyl sulfonyl] pyridine 1-oxide, m.p. 169-170°C. (IR Spectrum: -C≡CH 3180 $cm^{-1}$; $SO_2$, 1138, 1312 $cm^{-1}$).

## EXAMPLE V

To a well-stirred solution of 0.60 g (10.7 mmol) powdered potassium hydroxide, and 1.4 g (11.1 mmol) of dimethylsulfate in 20 mL of DMF was added 2.77 g (10 mmol) of 2-(2,5-dimethylphenylmethylsulfonyl) pyridine 1-oxide in one portion. The reaction temperature was maintained at 10°C for 30 minutes. The mixture was then poured into 300 mL of rapidly-stirred ice-cold water to afford a flocculent yellow precipitate. This solid was filtered off, washed with water, and air-dried to give 2.00 g of a mixture of 2-[1-2,5-dimethylphenyl)ethylsulfonyl] pyridine 1-oxide and starting material in a ratio of 85-15, respectively (determined by 'H-NMR). This represents a 69% recovery with an overall yield of 59%.

CLAIMS:

1. A method for alkylating substituted 2-sulfonyl pyridine 1-oxides characterised in that the method comprises the admixing of at least one compound having the formula

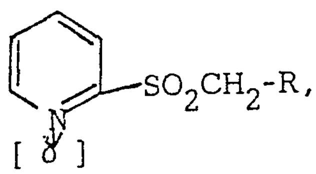

an alkylating agent, a polar aprotic solvent and a strong base to yield compounds having the formula

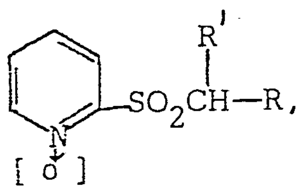

wherein: R is 2,2-dichlorocyclopropyl, 2,2-dichloro-1-methylcyclopropyl, naphthyl, methylnaphthyl, monosubstituted phenyl wherein the substituent is in the ortho position and is alkyl having 1 to 2 carbon atoms, halogen or nitro, or polysubstituted phenyl having from 2 to 5 substituents selected from alkyl having 1 to 2 carbon atoms, halogen, nitro, methoxy, ethoxy and methylenedioxy; and R' is methyl, ethyl, benzyl and substituted methyl, benzyl or naphthyl.

2. A method according to claim 1, characterised in that said polar aprotic solvent comprises at least one of N,N-dimethyl-formamide, N,N-dimethylacetamide and dimethylsulfoxide.

3. A method according to claim 1 or claim 2, characterised in that said strong base comprises at least one alkali or alkaline earth hydroxide.

4. A method according to claim 3, characterised in that said strong base is sodium hydroxide or potassium hydroxide.

5. A method according to any of the preceding claims, characterised in that said alkylating agent is an alkyl halide or sulfate or a substituted alkyl halide or sulfate.

6. A method according to claim 5, characterised in that said alkylating agent is a methyl, benzyl or allyl halide or a substituted methyl, benzyl or naphthyl halide.

7. A method according to claim 5, characterised in that said alkylating agent is methyl chloride, benzyl chloride, propargyl bromide, dimethyl sulfate, methyl iodide, ethyl bromide, 3-trifluoromethylbenzyl chloride, allyl chloride, vinyl sulfone, 1,3-dibromopropane, 1,6-dibromohexane or 2-chloromethylnaphthalene.

8. A method of alkylating 2-(2,5-dimethyl phenylmethyl sulfonyl) pyridine 1-oxide to form 2-/1-(2,5-dimethylphenyl) ethyl sulfonyl/ pyridine 1-oxide characterised in that the method comprises the admixing of 2-(2,5-dimethylphenylmethyl sulfonyl) pyridine 1-oxide, at least one of N,N-dimethylformamide, N,N-dimethylacetamide and dimethylsulfoxide, at least one alkali or alkaline earth hydroxide and at least one alkylating agent wherein the alkylating group is a methyl group.

9. A method according to claim 8, characterised in that said alkylating agent is a methyl halide or dimethyl sulfate.

10. A method of alkylating 2-(2,5-dimethylphenylmethyl sulfonyl) pyridine 1-oxide to form 2-/1-(2,5-dimethylphenyl) ethyl sulfonyl/ pyridine 1-oxide characterised in that the method comprises the

admixing of 2-(2,5-dimethylphenylmethylsulfonyl) pyridine 1-oxide, sodium hydroxide, N,N-dimethylformamide and at least one of methyl chloride and dimethylsulfate.

0028921

European Patent Office

**EUROPEAN SEARCH REPORT**

Application number

EP 80 30 3962

-2-

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| | tosylmethylisocyanide"<br>* Completely *<br>-- | | |
| A | CHEMICAL ABSTRACTS, vol. 90, 1979, page 474, no. 71716g<br>Columbus, Ohio, U.S.A.<br>A.A. GEVORKYAN et al.: "4,4-Dimethyl-1,3-dioxane as a solvent for the alkylation of active methylene compounds by alkyl halides in the presence of potassium or sodium hydroxide"<br>& ARM. KHIM. ZH. 1978, 31(8), 632-638<br>* Abstract *<br>-- | | TECHNICAL FIELDS SEARCHED (Int. Cl. 3) |
| A | CHEMICAL ABSTRACTS, vol. 88, 1978, page 567, no. 22101c<br>Columbus, Ohio, U.S.A.<br>S. ITO et al.: "A simple method for the alkylation of diethyl malonate"<br>& TOHOKU KOGYO GIJUTSU SHIKENSHO HOKOKU 1976, 7, 58<br>* Abstract *<br>---- | | |

0028921

European Patent Office

**EUROPEAN SEARCH REPORT**

Application number EP 80 30 3962

| Category | Citation of document with indication, where appropriate, of relevant passages | Relevant to claim | CLASSIFICATION OF THE APPLICATION (Int. Cl.3) |
|---|---|---|---|
| D,A | US - A - 3 960 542 (UNIROYAL INC.)<br>* Column 3, lines 49-62 *<br>-- | | C 07 D 213/89 |
| | SYNTHESIS, no. 6, 1979, pages 461-463<br>Stuttgart, DE.<br>J. GOLINSKI et al.: "Reactions of organic anions; XCIV. Catalytic two-phase alkylation of benzyl sulfones and sulfonamides"<br>* Completely *<br>-- | 1-5 | |
| | TETRAHEDRON LETTERS, no. 12, 1975, pages 1007-1010<br>Oxford, G.B.<br>K. KONDO et al.: "Sulfonyl carbanions in synthesis. I. A novel route to α,β-unsaturated carbonyl compounds"<br>* Completely *<br>-- | 1-5 | TECHNICAL FIELDS SEARCHED (Int. Cl.3)<br>C 07 D 213/89<br>213/71 |
| | TETRAHEDRON LETTERS, no. 48, 1977, pages 4229-4232<br>Oxford, G.B.<br>O. POSSEL et al.: "Tosylmethyl isocyanide employed in a novel synthesis of ketones. A new masked formaldehyde reagent"<br>* Completely *<br>-- | 1-5 | |
| | TETRAHEDRON LETTERS, no. 40, 1975, pages 3487-3488<br>Oxford, G.B.<br>A.M. VAN LEUSEN et al.: "Phase-transfer mono-alkylation of<br>./. | 1-5 | |

CATEGORY OF CITED DOCUMENTS

- X: particularly relevant
- A: technological background
- O: non-written disclosure
- P: intermediate document
- T: theory or principle underlying the invention
- E: conflicting application
- D: document cited in the application
- L: citation for other reasons
- &: member of the same patent family, corresponding document

X The present search report has been drawn up for all claims

| Place of search | Date of completion of the search | Examiner |
|---|---|---|
| The Hague | 05-02-1981 | VAN BIJLEN |

EPO Form 1503.1 06.78

BAD ORIGINAL